# EUROPÄISCHE PATENTANMELDUNG

(11) **EP 1 138 705 A1**
(43) Veröffentlichungstag der Anmeldung: **04.10.2001**
(21) Anmeldenummer: 01102470.0
(22) Anmeldetag: 03.02.2001
(51) Int. Cl.: C08G 18/02, C08G 18/18, C08G 18/79, C07D 251/34

(54) **Isocyanuratgruppenhaltige Polyisocyanate aus 1,4-Diisocyanato-2,2,6-trimethylcyclohexan, ein Verfahren zur Herstellung und Verwendung**

(30) Priorität: 30.03.2000 DE 10015890
(71) Anmelder: Degussa AG, 40474 Düsseldorf (DE)
(72) Erfinder: Spyrou, Emmanouil, Dr., 45772 Marl (DE); Pinske, Klaus, Dr., 45721 Haltern (DE); Kohlstruk, Stephan, Dr., 45770 Marl (DE)

(57) **Zusammenfassung**

Gegenstand der Erfindung sind isocyanuratgruppenhaltige Polyisocyanate aus 1,4-Diisocyanato-2,2,6-trimethylcyclohexan mit einem Anteil an Trimeren von mehr als 80 Gew.-% und einem NCO-Gehalt von mindestens 19,3 %.

## Beschreibung

Die Erfindung betrifft ein neues isocyanuratgruppenhaltiges Polyisocyanat aus 1,4-Diisocyanato-2,2,6-trimethylcyclohexan (TMCDI), ein Verfahren zur Herstellung und die Verwendung.

Aliphatische und cycloaliphatische Diisocyanate finden in hochwertigen licht- und wetterstabilen Polyurethanbeschichtungen breite Anwendung. Für viele Anwendungen, so z. B. für die Trimerisierung, ist es dabei von Vorteil, wenn die beiden Isocyanatgruppen unterschiedliche Reaktivitäten aufweisen. Der Anteil an unerwünschten Oligomeren (Pentamere, Heptamere, Nonamere usw.) wird durch diese so genannte Selektivität zurückgedrängt, die entstehenden Produkte (isocyanuratgruppenhaltige Polyisocyanate) weisen höhere NCO-Gehalt, niedrigere Viskositäten und bessere technische Verarbeitbarkeit auf.

Hexamethylendiisocyanat (HDI) weist keinerlei Selektivität auf, das entsprechende isocyanuratgruppenhaltige Polyisocyanat hat daher einen hohen Anteil an unerwünschten Oligomeren. Isophorondiisocyanat (IPDI) ist aufgrund der unterschiedlichen sterischen Umgebung der beiden Isocyanatgruppen dagegen selektiv, die Trimerisierung führt aber bei gleichen Verhältnissen zu geringeren Oligomergehalten von ca. 35 Gew.-% und ergibt einen Trimerenanteil von ca. 65 Gew.-%.

Aufgabe war es, ein trimeres Isocyanat zu finden mit einem höheren NCO-Gehalt und verbesserter, niedriger Viskosität. Die Aufgabe wurde durch das Trimerisierungsprodukt des TMCDI mit einem Anteil an Trimeren von mehr als 80 Gew.-% und einem NCO-Gehalt von mindestens 19,3 % gelöst.

Überraschend wurde gefunden, dass 1,4-Diisocyanato-2,2,6-trimethylcyclohexan (TMCDI), ein einfach zugängliches cycloaliphatisches Diisocyanat (J. Appl. Polym. Sci. 1994, *54(2)*, 207-218; EP 0 945 427 A1), sich im besonderen Maße für die Trimerisierung eignet. Der Oligomerengehalt der entstehenden isocyanuratgruppenhaltigen Polyisocyanate ist bei gleichem Umsatz deutlich niedriger als bei isocyanuratgruppenhaltigen Polyisocyanaten auf Basis IPDI, der NCO-Gehalt deutlich höher.

Gegenstand der Erfindung sind isocyanuratgruppenhaltige Polyisocyanate aus 1,4-Diisocyanato-2,2,6-trimethylcyclohexan mit einem Anteil an Trimeren von mehr als 80 Gew.-% und einem NCO-Gehalt von mindestens 19,3 %.

Ein weiterer Gegenstand der Erfindung ist ein Verfahren zur Herstellung isocyanuratgruppenhaltiger Polyisocyanate aus 1,4-Diisocyanato-2,2,6-trimethylcyclohexan mit einem Anteil an Trimeren von mehr als 80 Gew.-% und einem NCO-Gehalt von mindestens 19,3 % durch Umsetzung von TMCDI in Gegenwart mindestens eines Trimerisierungskatalysators bei 70 bis 150 °C.

1,4-Diisocyanato-2,2,6-trimethylcyclohexan, im Folgenden einfach als TMCDI bezeichnet, kann durch Trimerisierung in Gegenwart von geeigneten Katalysatoren in ein isocyanuratgruppenhaltiges Polyisocyanat überführt werden. Dazu wird TMCDI bei Raumtemperatur mit einem Katalysator versetzt und auf 130 °C aufgeheizt. Nach zwanzig Minuten ist die Reaktion in Abhängigkeit von der Katalysatormenge auf etwa 30-60 % Umsatz fortgeschritten und wird entweder thermisch oder durch Zugabe einer Säure abgebrochen. Überschüssiges Diisocyanat wird bei 150 °C und 0,1 mbar im Kurzwegverdampfer abdestilliert. Die so erhaltenen isocyanuratgruppenhaltigen Polyisocyanate haben einen Oligomerenanteil von 8-20 Gew.-% (Trimeranteil 92-80 Gew.-%) und eine NCO-Gehalt von 19,3-20,0 %.

Geeignete Trimerisierungskatalysatoren sind z.B. quartäre Ammoniumcarboxylate, Alkali- und Erdalkalisalze von Carbonsäuren, Trialkylphosphine oder quarternäre Ammoniumcarbonate oder Aminosilanverbindungen. Besonders geeignet ist z.B. Trioctylmethylammonium-2-ethylhexanoat, das aus dem entsprechenden Chlorid nach dem in US 5,691,440 näher beschriebenen Verfahren hergestellt werden kann.

Isocyanuratgruppenhaltige Polyisocyanate auf Basis TMCDI stellen, eventuell in blockierter Form, wertvolle Ausgangsmaterialen zur Herstellung von Polyurethankunststoffen nach dem Isocyanat-Polyadditionsverfahren, als Lackrohstoffe und insbesondere zur Herstellung von Ein- oder Zweikomponenten-Polyurethanlacken dar.

Geeignete Blockierungsmittel sind in der Isocyanatchemie bekannt, z. B. Acetessigsäureethylester, Diisopropylamin, Methylethylketoxim, Malonsäurediethylester, ε-Caprolactam, 1,2,4-Triazol, 3,5-Dimethylpyrazol.

Die folgenden Beispiele sollen die Erfindung erläutern aber nicht einschränken:
Herstellung eines isocyanuratgruppenhaltigen Polyisocyanates aus 1,4-Diisocyanato-2,2,6-trimethylcyclohexan
292 g 1,4-Diisocyanato-2,2,6-trimethylcyclohexan wurden vorgelegt, mit 1,46 g Trioctylmethylammonium-2-ethylhexanoat versetzt und unter Schutzgas und Rühren auf 130 °C aufgeheizt. Nach 20 Min. wurde die Temperatur kurzzeitig auf 150 °C erhöht und danach abgekühlt (NCO-Gehalt: 31,1%, 45% Umsatz). Überschüssiges TMCDI ließ sich bei 150 °C und 0,1 mbar am Kurzwegverdampfer abtrennen. Das entmonomerisierte Produkt wies einen NCO-Gehalt von 19,8 % auf und einen Oligomerenanteil von 8 % (Trimeranteil 92 %) (Gelpermeationschromatographie (GPC), Superfluidchromatographie (SFC)) auf.

Nicht erfindungsgemäßes Vergleichsbeispiel:
Herstellung eines isocyanuratgruppenhaltigen Polyisocyanates aus Isophorondiisocyanat
286 g Isophorondiisocyanat wurden vorgelegt, mit 0,25 g Trioctylmethylammonium-2-ethylhexanoat versetzt und unter Schutzgas und Rühren auf 70 °C aufgeheizt. Nach dieser Initiierung durchlief die Temperatur der exothermen Reaktion ein Maximum und fiel danach wieder ab (NCO-Gehalt: 29,1 %, 46 % Umsatz). Überschüssiges IPDI ließ sich bei 150 °C und 0,1 mbar am Kurzwegverdampfer abtrennen. Das entmonomerisierte Produkt wies einen NCO-Gehalt von 17,4 % auf und einen Oligomerenanteil von 33 % (Trimeranteil 67 %) (GPC, SFC) auf.

## Patentansprüche

1. Isocyanuratgruppenhaltige Polyisocyanate aus 1,4-Diisocyanato-2,2,6-trimethylcyclohexan, mit einem Anteil an Trimeren von mehr als 80 Gew.-% und einem NCO-Gehalt von mindestens 19,3 %.

2. Isocyanuratgruppenhaltige Polyisocyanate nach Anspruch 1,
**dadurch gekennzeichnet,**
**dass** der Anteil an Trimeren größer 90 Gew.-% beträgt, mit einem NCO-Gehalt von mindestens 19,6 %.

3. Isocyanuratgruppenhaltige Polyisocyanate nach Anspruch 1 oder 2,
**dadurch gekennzeichnet,**
**dass** es blockiert ist.

4. Isocyanuratgruppenhaltige Polyisocyanate nach Anspruch 3,
**dadurch gekennzeichnet,**
**dass** als Blockierungsmittel Acetessigsäureethylester, Diisopropylamin, Methylethylketoxim, Malonsäurediethylester, ε-Caprolactam, 1,2,4-Triazol oder 3,5-Dimethylpyrazol oder Gemische dieser eingesetzt werden.

5. Verfahren zur Herstellung isocyanuratgruppenhaltiger Polyisocyanate aus 1,4-Diisocyanato-2,2,6-trimethylcyclohexan mit einem Anteil an Trimeren von mehr als 80 Gew.-% und einem NCO-Gehalt von 19,3 % durch Umsetzung von TMCDI in Gegenwart mindestens eines Trimerisierungskatalysators bei 70 bis 150 °C.

6. Verfahren nach Anspruch 5,
**dadurch gekennzeichnet,**
**dass** als Katalysator quartäre Ammoniumcarboxylate, Alkali- und Erdalkalisalze von Carbonsäuren, Trialkylphosphine, quarternäre Ammoniumcarbonate oder Aminosilanverbindungen eingesetzt werden.

7. Verfahren nach Anspruch 6,
**dadurch gekennzeichnet,**
**dass** als Katalysator Trioctylmethylammonium-2-ethylhexanoat eingesetzt wird.

8. Verwendung des Trimerisierungsprodukts des TMCDI mit einem Anteil an Trimeren von mehr als 80 Gew.-% und einem NCO-Gehalt von mindestens 19,3 % als Lackrohstoffe.

9. Verwendung nach Anspruch 8 zur Herstellung von Ein- oder Zweikomponenten-Polyurethanlacken.
